# EUROPÄISCHE PATENTSCHRIFT

(11) **EP 1 915 962 B1**
(45) Veröffentlichungstag und Bekanntmachung des Hinweises auf die Patenterteilung: **03.12.2008**
(21) Anmeldenummer: 06022395.5
(22) Anmeldetag: 26.10.2006
(51) Int. Cl.: A61B 19/00

(54) **Integriertes medizinisches Trackingsystem**
Integrated medical trackingsystem
Système médical integré de localisation et de suivi

(43) Veröffentlichungstag der Anmeldung: 30.04.2008
(73) Patentinhaber: BrainLAB AG, 85622 Feldkirchen (DE)
(72) Erfinder: Goldbach, Günter, 85457 Wörth/Wilfing (DE)
(74) Vertreter: Schwabe - Sandmair - Marx

(56) Entgegenhaltungen:
- EP-A- 1 216 651
- DE-U1- 20 321 068
- US-A- 5 295 483
- US-A1- 2001 034 530
- US-A1- 2004 010 190
- US-A1- 2004 105 086

## Beschreibung

Die Erfindung betrifft ein integriertes medizintechnisches Trackingsystem. Herkömmliche medizintechnische Trackingsysteme weisen hauptsächlich eine Trackingeinheit auf, die ein Bildaufnahme- und Bildsignalverarbeitungssystem umfasst. Diese Technology basiert auf einer Hardware, die digitale Signalprozessoren (DSPs) und sogenannte Field Programmable Gate Arrays (FPGAs) umfasst. Als Ausgangssignal liefert ein solcher herkömmliches Trackingsystem lediglich Koordinaten von erkannten Punkten zum Beispiel Markern; eine weitere Signalverarbeitung findet nicht statt. Solche herkömmlichen Trackingsysteme sind beispielsweise aus der US 5,817,105 und der US 5,828,770 bekannt.

Aus der US 2004/0,105,086 A1 ist ein medizintechnisches Trackingsystem bekannt, bei welchem eine Rechnereinheit zusammen mit einer Anzeigeeinheit und Sensoren auf einer rollbaren Plattform angeordnet sind.

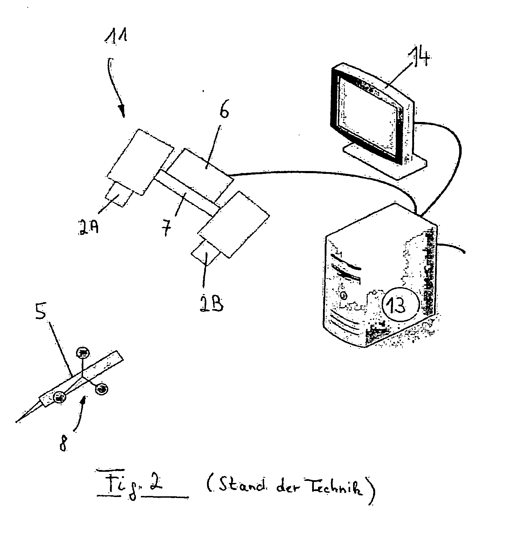

Die beiliegende Figur 2 soll nun dazu verwendet werden, um darzustellen, wie Trackingsysteme bisher grundsätzlich gestaltet und eingesetzt wurden. Zu sehen ist in der Figur 2 ein herkömmliches Trackingsystem, das mit dem Bezugszeichen 11 versehen worden ist. Das Trackingsystem umfasst eine Datenerfassungseinheit mit typischerweise zwei oder mehr Sensoranordnungen (Kameras), zur Erfassung von Informationen über die Raumposition getrackter/navigierter Objekte (Marker) und/oder Instrumente. Die Kameras sind mit den Bezugszeichen 2A und 2B gekennzeichnet und mit einer Strebe 7 verbunden. Das Trackingsystem 11 umfasst Bildverarbeitungseinrichtungen, wie die oben genannten DSPs und FPGAs sowie eine Kalibrierungswert-Abspeicherungseinrichtung. Ganz allgemein liefert dieses Trackingsystem 11 die Daten zur weiteren Verarbeitung auf einer von ihm entfernten Computereinheit, die das Bezugszeichen 13 trägt. Diese Computereinheit 13 sorgt für die weitere Datenverarbeitung, die anwendungsspezifische Programmierung und die Datenspeicherung. In ihr werden Funktionen wie die grafische Benutzerschnittstellen-Programmierung, Schnittstellen für andere Vorrichtungen, wie zum Beispiel Mikroskope, C-Bögen, etc. realisiert. Die Visualisierung der getrackten Objekte und Instrumente wird gewöhnlich auf einem angebundenen Bildschirm 14 durchgeführt, der auch Eingabe- und Steuerungsfunktionen haben kann.

In der Figur 2 ist noch das Instrument 5 dargestellt, das mit Hilfe seines Referenzmarker-Adaperts 8 getrackt bzw. navigiert wird.

Problematisch bei solchen herkömmlichen Systemen ist das relativ komplizierte und raumgreifende Setup mit der Anzahl separater zu verwendender Geräte. Die Handhabung und Verbindung der Geräte untereinander ist relativ kompliziert.

Die Erfindung hat es sich zur Aufgabe gemacht, in dieser Hinsicht Abhilfe zu schaffen. Insbesondere soll ein gut handhabbares und unkompliziert verwendbares Trackingsystem geschaffen werden.

Diese Aufgabe wird erfindungsgemäß durch ein medizintechnisches Trackingsystem gemäß dem Anspruch 1 gelöst. Die Unteransprüche definieren bevorzugte Ausführungsformen der Erfindung.

Erfindungsgemäß wird im Trackingsystem eine programmierbare Computerplattform vorgesehen, und zwar eine die mit der Trackingeinheit integriert ist. Mit anderen Worten wird aus einer relativ unintelligenten Koordinaten-Erfassungsmaschine eine intelligente und beeinflussbare, integrale Einheit gemacht, denn mit Hilfe der programmierbaren Computerplattform können Funktionen, die bisher im Rahmen der nötigen technischen Navigation außerhalb des Trackingsystems stattgefunden haben, in das Trackingsystem integriert werden. Hierbei ist es von besonderem Vorteil, dass die Miniaturisierung von Computerplattformen relativ stark fortgeschritten ist und durch ihre Einbindung in ein Trackingsystem somit integrierte Hardwarelösungen für das Tracking geschaffen werden können, die insgesamt noch eine sehr geringe Größe und deshalb auch eine gute Handhabbarkeit aufweisen.

Wenn im Weiteren von Trackingsystemen gesprochen wird, wird des Öfteren auf optische Trackingsysteme Bezug genommen, die natürlich ein Teil der vorliegenden Erfindung sind. Es ist aber natürlich ebenso möglich, die vorliegende Erfindung auf andere Arten von Trackingsystemen medizintechnischer Natur anzuwenden, beispielsweise auf Magnettrackingsysteme.

Es ist im Rahmen der Erfindung möglich, die programmierbare Computerplattform mit einer wichtigen, einigen wichtigen oder mit einer großen Anzahl von Funktionen auszustatten, die früher durch externe Navigations-Hardware übernommen wurden. Während schon wenige übertragene Fähigkeiten das Trackingsystem selbst universeller einsetzbar machen, kann die Übertragung einer großen Vielzahl von Aufgaben bis zur völligen Eliminierung einer zusätzlichen Datenverarbeitungs- und Speichereinheit führen. Jeder Grad von Übertragungen mag für gewisse Anwendungsfälle seine größten Vorzüge haben; wichtig ist, dass durch die vorliegende Erfindung schon die Möglichkeit geschaffen wird, solche früher extern bewältigten Funktionen direkt im Trackingsystem durchzuführen. Die weiteren Vorteile liegen im stark verbesserten ergonomischen Handling und stark verbesserten Aufbaumöglichkeiten (Setup), der Eliminierung von mehreren externen Kabelverbindungen, der Möglichkeit, ein stabiles Systemgehäuse für das gesamte Trackingsystem bereitzustellen und der Optimierung der Wartungs- und Service-Fähigkeiten.

Das Trackingsystem ist als Baueinheit realisiert, welche die Trackingeinheit und die Computerplattform umfasst. Die Trackingeinheit und die Computerplattform sind in einem, insbesondere einem einzigen Gehäuse untergebracht.

Es ist im Rahmen der Erfindung möglich, dass die programmierbare Computerplattform mindestens einen Teil einer medizintechnischen Navigationseinheit umfasst. Mit anderen Worten werden Navigationsaufgaben auf die im Trackingsystem integrierte Computerplattform übertragen.

Dem Trackingsystem, insbesondere der Computerplattform bzw. der Navigationseinheit kann eine Mehrzweck-Breitband-Schnittstelle zugeordnet sein, und zwar für die Datenübertragung mit Instrumenten oder Geräten, die in das Tracking einbezogen sind. Solche Instrumente oder Geräte können Mikroskope, C-Bogen-Röntgengeräte oder ähnliche Geräte im medizintechnischen Umfeld sein, die bei einer medizintechnischen Navigation mit erfasst werden. Bei einer weiteren Ausführungsform der Erfindung ist dem Trackingsystem, insbesondere der Computerplattform bzw. der Navigationseinheit, ein Massenspeichergerät, insbesondere ein auswechselbares Massenspeichergerät zugeordnet, wobei das Massenspeichergerät zum Beispiel eine Festplatte sein kann. Es kann sich aber auch um beschreibbare Datenträger wie CDs oder DVDs oder große Chip-Speicher handeln.

Es ist im Rahmen der Erfindung möglich, dem Trackingsystem, insbesondere der Computerplattform bzw. der Navigationseinheit, eine Fernbedienung zuzuordnen, und gemäß einer anderen Ausführungsform kann dem Trackingsystem oder einer der oben beschriebenen Komponenten eine grafische Benutzerschnittstelle bzw. Anzeigeeinrichtung zugeordnet sein, die insbesondere abnehmbar bzw. durch Kabel oder kabellos mit dem Trackingsystem verbunden ist. Weitere dem Trackingsystem zuordnungsfähige Einrichtungen sind beispielsweise eine Beleuchtungseinrichtung für das Operationsfeld, insbesondere eine Markerbeleuchtungseinrichtung, oder eine Steuerungsschnittstelle für die Steuerungseinrichtung zum Steuern des Trackingsystems.

Wie schon oben beschrieben, besteht erfindungsgemäß die Möglichkeit, der programmierbaren Computerplattform bzw. der Navigationseinheit einzelne oder mehrere Funktionen zuzuordnen. Diese Funktionen können sein:
- eine Synchron-Bilderfassungsfunktion,
- eine Bildverarbeitungsfunktion,
- eine Markererkennungs- und/oder Markerverfolgungsfunktion,
- eine Instrumentenerkennungs- und/oder Instrumentenverfolgungsfunktion,
- eine Speicherfunktion, insbesondere für anwendungsspezifische Informationen, Patienteninformationen, aktualisierbare Trackingsystem-Arbeitsinformationen (Upgrades) oder Informationen über verwendete medizintechnische Apparaturen, Instrumente oder Geräte,
- eine Remote-Login-Funktion,
- eine Funktion zur graphischen Visualisierung und/oder zum graphischen Einblenden von Instrumenten bzw. Instrumentenformen,
- eine Funktion zur Umsetzung bzw. Durchführung oder Unterstützung von anwendungsspezifischen Aufgaben.

Die Kombination von Bilderfassung, Bildverarbeitung, Mensch-Maschine-Schnittstelle und Visualisierungseinrichtung basierend auf einer programmierbaren Computerplattform ist speziell für stereoskopische Kamera-Setups von Vorteil, wo es wichtig ist, dass Bilder aus zwei oder mehr Datenerfassungseinheiten gleichzeitig verarbeitet werden.

Einzelne und gesonderte bzw. unabhängig voneinander existierende Aspekte der vorliegenden Erfindung sind:
- ein Trackingsystem mit einer integrierten programmierbaren Computerplattform;
- ein Trackingsystem mit einer integrierten Mehrzweck-(Breitband-)Schnittstelle, die nicht auf Punkt-zu-Punkt-Verbindungen beschränkt ist;
- ein Trackingsystem mit einer integrierten Massenspeichervorrichtung:

- ein Trackingsystem mit einer Fernsteuerungsfähigkeit.

Hauptsächliche technischen Änderungen gegenüber herkömmlichen Systemen bestehen darin, dass die erfindungsgemäßen integrierten Trackingsysteme eine fortgeschrittene Bildverarbeitung und Kommunikation gestatten, indem ein komplettes programmierbares Computersystem integriert wird, mit Massenspeichervorrichtungen, integriertem RAM und generischen (Hochgeschwindigkeits-) Schnittstellen zur Anbindung anderer externer Vorrichtungen, zur Vorbereitung für eine Fernsteuerung, für Serviceaufgaben und Updatefunktionen. Anwenderspezifische Software könnte unter Verwendung der MehrzweckSchnittstelle auf das Trackingsystem heruntergeladen werden. Die Möglichkeit, eine geringe Größe und ein geringes Gewicht für die integrierte Einheit zur Verfügung zu stellen, gestattet neue Setup-Möglichkeiten und eliminiert den Bedarf an einem zusätzlichen Computer zur Steuerung und Verarbeitung der Sensordateninformationen. Eine Vielzahl von Kabelverbindungen kann eliminiert werden, und die Anzeige- und Steuerungsvorrichtungen können sehr flexibel ausgestaltet und per Software konfiguriert werden, um das integrierte Trackingsystem zu steuern. Eine Fernsteuerung des integrierten Trackingsystems mit Hilfe von sehr kleinen und unkomplizierten Geräten wie zum Beispiel PDAs, Laptops aber auch zum Beispiel Mobiltelefonen oder mit anderen Endgeräten wird möglich.

Die Erfindung wird im Weiteren nochmals anhand einer Ausführungsform näher beschrieben. Sie kann alle hierin beschriebenen Merkmale einzeln sowie in jedweder sinnvollen Kombination umfassen.

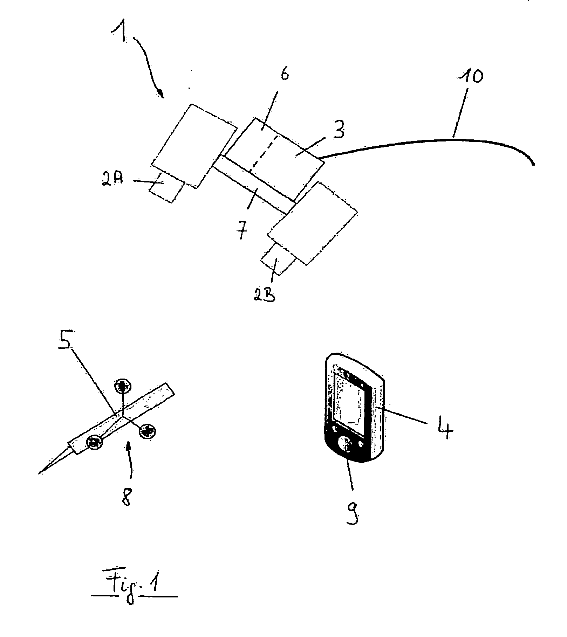

Die Figur 1 zeigt eine schematische Darstellung einer erfindungsgemäßen Trackingsystem-Ausführung.

Das in Figur 1 gezeigte Trackingsystem 1 umfasst wiederum zwei Kameras 2A, 2B, die mit der Strebe 7 verbunden sind und so stereoskopische Bilder, beispielsweise eines Instruments 5 und dessen Referenzmarker-Anordnung 8 aufnehmen. Es hat natürlich auch die Fähigkeiten der Bildverarbeitung, und dies ist durch den Teil des Trackingsystems dargestellt, der in Figur 1 (so wie vorher in Figur 2) mit dem Bezugszeichen 6 gekennzeichnet ist.

Zusätzlich umfasst das Trackingsystem 1 aber noch die programmierbare Computerplattform 3, ein Computersystem, und deshalb sind die wichtigen Tracking-Funktionseinheiten einschließlich der Programmierung und der Massenspeichereinrichtung vollständig in einer Einheit integriert. Anders als beim System nach Figur 2 ist beim System nach Figur 1 eine generische Schnittstelle 10 mit großer Bandbreite zur Verfügung gestellt, die als einzige und Mehrzweckschnittstelle dienen kann und damit eine komplizierte Verkabelung unnötig macht. Mit Hilfe dieser Schnittstelle 10 können entfernt angeordnete Geräte, wie zum Beispiel eine Anzeigevorrichtung 4 sowie andere medizintechnische Ausstattungen wie C-Bögen, Mikroskope etc. angebunden werden. Ferner dient diese Schnittstelle 10 dazu, das integrierte Trackingsystem 1 direkt mit einem Datenspeicherungs- und Datenbank-Netzwerk zu verbinden. Eine bevorzugte Ausführung der Schnittstelle 10 ist beispielsweise eine Gigabit-Ethernet-Schnittstelle, eine W-LAN-Schnittstelle oder eine Hochgeschwindigkeits-Faseroptik-Schnittstelle. Ein weiteres spezielles Merkmal dieser Schnittstelle ist, dass sie nicht länger eine Punkt-zu-Punkt-Verbindung mit einem Hauptcomputer sein muss, sondern eine Verbindung mehrerer Vorrichtungen über eine physikalische Schnittstelleneinheit gestattet.

Je nach dem Typ des Sensorsystems 2A, 2B, kann das Trackingsystem Beleuchtungseinrichtungen umfassen, beispielsweise Infrarot-Beleuchtungseinrichtungen für Marker, wie die Marker an der Referenzanordnung 8 des Instruments 5. Speziell bei Systemen mit kabellosen Instrumenten ist eine solche Beleuchtungsvorrichtung am Trackingsystem typischerweise notwendig.

Schon durch ein kleines Gerät, das in Figur 1 das Bezugszeichen 4 trägt und eine grafische Benutzerschnittstelle und Systemsteuerung darstellt, wird das Trackingsystem 1 zu einem verwendungsfähigen medizintechnischen Navigationssystem. Das Gerät 4 hat einen kleinen Bildschirm (Grafikausgabe) und erfüllt eine Vielzahl von Bildausgabe- und Steuerungsfunktionen für das integrierte Trackingsystem 1, beispielsweise über eine W-LAN-Verbindung. Mögliche Umsetzungen für das Gerät 4 zur Steuerung des Trackingsystems 1 sind ein PDA (Handcomputer), ein Tablet-PC, ein Laptop-PC, eine Remote-Terminaleinheit mit Anzeige und Tastatur, oder in bestimmten Fällen möglicherweise sogar ein Mobiltelefon. Das Gerät 4 kann verschiedene Eingabevorrichtungen umfassen, wie zum Beispiel einen Touch-Screen, Knöpfe 9 aber auch Mikrophone für eine Sprachsteuerung, eine Bewegungserfassung oder andere Eingabemittel. Das Gerät 4 kann eine Software-Programmierfähigkeit aufweisen, kann aber auch ein einfaches vorkonfiguriertes Steuerungsgerät sein.

Die Erfindung, beispielsweise in der gezeigten Ausführungsform der Figur 1, vereinfacht den gesamten Aufbau, verringert die Anzahl der benötigten Komponenten und stellt ein vollständig integriertes Trackingsystem in einem einzigen Gehäuse zur Verfügung. Sie macht auf diese Weise das medizintechnische Tracking und die medizintechnische Navigation flexibel und einfach.

## Patentansprüche

1. Medizintechnisches Trackingsystem (1) mit einer Trackingeinheit, die ein Bildaufnahme- und Bildsignalverarbeitungssystem (2, 6) umfasst, wobei im Trackingsystem (1) integriert in und/oder an der Trackingeinheit (2, 6, 7) eine programmierbare Computerplattform (3) vorgesehen ist, **dadurch gekennzeichnet, dass** das Trackingsystem (1) als Bauteil realisiert ist, welche die Trackingeinheit (2, 6, 7) und die Computerplattform (3) umfasst, die in einem Gehäuse untergebracht sind.

2. Trackingsystem nach Anspruch 1, **dadurch gekennzeichnet, dass** die programmierbare Computerplattform (3) mindestens einen Teil einer medizintechnischen Navigationseinheit umfasst.

3. Trackingsystem nach Anspruch 1 oder 2, **dadurch gekennzeichnet, dass** dem Trackingsystem, insbesondere der Computerplattform bzw. der Navigationseinheit, eine Mehrzweck-Breitband-Schnittstelle (10) zugeordnet ist, für die Datenübertragung mit Instrumenten oder Geräten, die in das Tracking einbezogen sind.

4. Trackingsystem nach einem der Ansprüche 1 bis 3, **dadurch gekennzeichnet, dass** dem Trackingsystem, insbesondere der Computerplattform bzw. der Navigationseinheit, ein Massenspeichergerät, insbesondere ein auswechselbares Massenspeichergerät zugeordnet ist, wobei das Massenspeichergerät z. B. eine Festplatte ist.

5. Trackingsystem nach einem der Ansprüche 1 bis 4, **dadurch gekennzeichnet, dass** dem Trackingsystem, insbesondere der Computerplattform bzw. der Navigationseinheit, eine Fernbedienung zugeordnet ist.

6. Trackingsystem nach einem der Ansprüche 1 bis 5, **dadurch gekennzeichnet, dass** dem Trackingsystem, insbesondere der Computerplattform bzw. der Navigationseinheit, eine graphische Benutzerschnittstelle bzw. Anzeigeeinrichtung zugeordnet ist, die insbesondere abnehmbar bzw. durch Kabel oder kabellos mit dem Trackingsystem verbunden ist.

7. Trackingsystem nach einem der Ansprüche 1 bis 6, **dadurch gekennzeichnet, dass** dem Trackingsystem mindestens eine der folgenden Einrichtungen zugeordnet:
- eine Beleuchtungseinrichtung für das Operationsfeld, insbesondere eine Markerbeleuchtungseinrichtung,
- eine Steuerungsschnittstelle für eine Steuerungseinrichtung zum Steuern des Trackingsystems.

8. Trackingsystem nach einem der Ansprüche 1 bis 7, **dadurch gekennzeichnet, dass** die programmierbare Computerplattform bzw. die Navigationseinheit (3) mindestens eine der folgenden Funktionen aufweist:
- eine Synchron-Bilderfassungsfunktion,
- eine Bildverarbeitungsfunktion,
- eine Markererkennungs- und/oder Markerverfolgungsfunktion,
- eine Instrumentenerkennungs- und/oder Instrumentenverfolgungsfunktion,
- eine Speicherfunktion, insbesondere für anwendungsspezifische Informationen, Patienteninformationen, aktualisierbare Trackingsystem-Arbeitsinformationen (Upgrades) oder Informationen über verwendete medizintechnische Apparaturen, Instrumente oder Geräte,
- eine Remote-Login-Funktion,
- eine Funktion zur graphischen Visualisierung und/oder zum graphischen Einblenden von Instrumenten bzw. Instrumentenformen,
- eine Funktion zur Umsetzung bzw. Durchführung oder Unterstützung von anwendungsspezifischen Aufgaben.

## Claims

1. A medical tracking system (1) comprising a tracking unit which includes an image recording and image signal processing system (2, 6), wherein a programmable computer platform (3) is provided in the tracking system (1), integrated in and/or on the tracking unit (2, 6, 7), **characterised in that** the tracking system (1) is realised as an assembly which includes the tracking unit (2, 6, 7) and the computer platform (3), which are accommodated in a casing.

2. The tracking system according to claim 1, **characterised in that** the programmable computer platform (3) includes at least a part of a medical navigation unit.

3. The tracking system according to claim 1 or 2, **characterised in that** a multi-purpose broadband interface (10) is assigned to the tracking system, in particular to the computer platform or navigation unit, for data transfer with instruments or devices which are involved in tracking.

4. The tracking system according to any one of claims 1 to 3, **characterised in that** a mass storage device, in particular an exchangeable mass storage device, is assigned to the tracking system, in particular to the computer platform or navigation unit, wherein the mass storage device is for example a hard drive.

5. The tracking system according to any one of claims 1 to 4, **characterised in that** a remote control device is assigned to the tracking system, in particular to the computer platform or navigation unit.

6. The tracking system according to any one of claims 1 to 5, **characterised in that** a graphic user interface or display means which is connected to the tracking system, in particular detachably and/or via cables or wirelessly, is assigned to the tracking system, in particular to the computer platform or navigation unit.

7. The tracking system according to any one of claims 1 to 6, **characterised in that** at least one of the following means is assigned to the tracking system:
- an illumination means for the operating field, in particular a marker illumination means;
- a control interface for a control means, for controlling the tracking system.

8. The tracking system according to any one of claims 1 to 7, **characterised in that** the programmable computer platform or navigation unit (3) exhibits at least one of the following functions:
- a synchronous image acquisition function;
- an image processing function;
- a marker identification and/or tracking function;
- an instrument identification and/or tracking function;
- a storage function, in particular for application-specific information, patient information, updatable tracking system operational information (updates) or information concerning medical apparatus, instruments or devices used;
- a remote login function;
- a function for graphically visualising and/or graphically superimposing instruments or instrument shapes;
- a function for implementing or performing or assisting application-specific tasks.

## Revendications

1. Système de poursuite médical (1) avec une unité de poursuite qui comprend un système de prise de vues et de traitement de signaux d'image (2, 6), dans le système de poursuite (1) étant prévue, dans et/ou sur l'unité de poursuite (2, 6, 7), une plateforme d'ordinateur (3) programmable, **caractérisé en ce que** le système de poursuite (1) est réalisé sous la forme d'un composant qui comprend l'unité de poursuite (2, 6, 7) et la plateforme d'ordinateur (3), lesquelles sont logées dans un boîtier.

2. Système de poursuite selon la revendication 1, **caractérisé en ce que** la plateforme d'ordinateur (3) programmable comprend au moins une partie d'une unité de navigation médicale.

3. Système de poursuite selon l'une quelconque des revendications 1 ou 2, **caractérisé en ce qu'**au système de poursuite, en particulier à la plateforme d'ordinateur ou à l'unité de navigation, est associée une interface à bande large multifonctionnelle (10), pour la transmission de données avec des instruments ou appareils qui sont intégrés dans la poursuite.

4. Système de poursuite selon l'une quelconque des revendications 1 à 3, **caractérisé en ce qu'**au système de poursuite, en particulier à la plateforme d'ordinateur ou à l'unité de navigation, est associée une mémoire de masse, en particulier une mémoire de masse échangeable, la mémoire de masse échangeable étant par exemple un disque dur.

5. Système de poursuite selon l'une quelconque des revendications 1 à 4, **caractérisé en ce qu'**au système de poursuite, en particulier à la plateforme d'ordinateur ou à l'unité de navigation, est associée une télécommande.

6. Système de poursuite selon l'une quelconque des revendications 1 à 5, **caractérisé en ce qu'**au système de poursuite, en particulier à la plateforme d'ordinateur ou à l'unité de navigation, est associée une interface utilisateur graphique ou dispositif d'affichage qui est relié en particulier de manière amovible ou par câble ou sans câble au système de poursuite.

7. Système de poursuite selon l'une quelconque des revendications 1 à 6, **caractérisé en ce qu'**au système de poursuite est associé au moins l'un des dispositifs suivantes :
- un dispositif d'éclairage pour le champ opératoire, en particulier un dispositif d'éclairage de repère,
- une interface de commande pour un dispositif de commande afin de commander le système de poursuite.

8. Système de poursuite selon l'une quelconque des revendications 1 à 7, **caractérisé en ce que** la plateforme d'ordinateur programmable ou l'unité de navigation (3) présente au moins l'une des fonctions suivantes :
- une fonction de relevé d'image synchrone,
- une fonction de traitement d'image,
- une fonction de reconnaissance de repère et/ou de poursuite de repère,
- une fonction de reconnaissance d'instrument et/ou de poursuite d'instrument,
- une fonction de mémoire, en particulier pour des informations spécifiques de l'application, des informations relatives au patient, des informations de travail du système de poursuite actualisables (upgrades) ou des informations relatives à des appareillages, instruments ou dispositifs médicaux utilisés,
- une fonction d'enregistrement à distance,
- une fonction pour la visualisation graphique et/ou pour la superposition graphique d'instruments ou de formes d'instrument,
- une fonction pour la réalisation ou l'exécution ou le soutien à des tâches spécifiques de l'application.
